(19) Europäisches Patentamt
European Patent Office
Office européen des brevets

(11) EP 1 137 678 B1

(12) **EUROPÄISCHE PATENTSCHRIFT**

(45) Veröffentlichungstag und Bekanntmachung des Hinweises auf die Patenterteilung:
**06.05.2004 Patentblatt 2004/19**

(21) Anmeldenummer: **99958065.7**

(22) Anmeldetag: **18.11.1999**

(51) Int Cl.7: **C08F 20/04**, A61L 15/18, A61L 15/60

(86) Internationale Anmeldenummer:
**PCT/EP1999/008850**

(87) Internationale Veröffentlichungsnummer:
**WO 2000/031157 (02.06.2000 Gazette 2000/22)**

(54) **WÄSSRIGE FLÜSSIGKEITEN ABSORBIERENDE HYDROGELE**
HYDROGELS ABSORBING AQUEOUS FLUIDS
HYDROGELS ABSORBANT DES LIQUIDES AQUEUX

(84) Benannte Vertragsstaaten:
**AT BE CH CY DE DK ES FI FR GB GR IE IT LI LU MC NL PT SE**

(30) Priorität: **26.11.1998 DE 19854575**

(43) Veröffentlichungstag der Anmeldung:
**04.10.2001 Patentblatt 2001/40**

(73) Patentinhaber: **BASF AKTIENGESELLSCHAFT**
**67056 Ludwigshafen (DE)**

(72) Erfinder:
- **DANIEL, Thomas**
  **D-67165 Waldsee (DE)**
- **RIEGEL, Ulrich**
  **D-60386 Frankfurt (DE)**
- **WEISMANTEL, Matthias**
  **D-63637 Jossgrund (DE)**
- **HERFERT, Norbert**
  **63674 Altenstadt (DE)**
- **ENGELHARDT, Friedrich**
  **Chesapeake, VA 23320 (US)**

(56) Entgegenhaltungen:
**EP-A- 0 471 595**
**EP-A- 0 629 411**
**WO-A-93/17066**
**CH-A- 185 779**
**US-A- 5 032 628**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 137 678 B1

## Beschreibung

Die vorliegende Erfindung betrifft Hydrogele, hergestellt unter Verwendung von Salzen der Kieselsäure, ein Verfahren zu ihrer Herstellung sowie ihre Verwendung zur Absorption wäßriger Flüssigkeiten.

Es handelt sich bei den erfindungsgemäßen wäßrigen Flüssigkeiten absorbierenden Hydrogelen um wasserunlösliche, Carboxylatgruppen enthaltende Polymere, welche eine Polysilikatmatrix enthalten und in der Lage sind unter Quellung und Ausbildung von Hydrogelen wäßrige Flüssigkeiten und Körperflüssigkeiten, wie z.B. Urin oder Blut aufzunehmen und die absorbierten Flüssigkeitsmengen unter einem bestimmten Druck zurückzuhalten.

Um Polymere herzustellen, welche Hydrogele mit besonders hoher Flüssigkeitsaufnahmekapazität, hoher Gelstärke sowie hohem Aufnahmevermögen unter Druck bilden, kann man die Polymerisatpartikel einer nachträglichen Oberflächenbehandlung, der Nachvernetzung, unterwerfen.

Bevorzugt werden zur Nachvernetzung solche Substanzen eingesetzt, welche zwei oder mehrere Gruppen enthalten, die mit den Carboxylgruppen der hydrophilen Polymeren kovalente Bindungen ausbilden können, siehe EP-A-0 349 240. Man bezeichnet solche Substanzen als Vernetzungsmittel oder Vernetzer.

Als Vernetzungsmittel sind Polyglycidylether, Haloepoxiverbindungen, Polyole, Polyamine oder Polyisocyanate bekannt. Des weiteren werden in der DE-A-3 314 019, EP-A-0 317 106 und DE-A-3 737 196 polyfunktionelle Aziridinverbindungen, Alkyl-di-(tri)halogenide und öllösliche Polyepoxidverbindungen als Vernetzer genannt.

Nach der DE-A-4 020 780 wird eine verbesserte Absorption unter Druck durch oberflâchenvernetzende Behandlung eines Polymeren mit 0,1 bis 5 Gew.-% Alkylencarbonat erreicht.

Die nachträgliche Zugabe von feinteiligen amorphen Polykieselsäuren (Silica) wie AERIL® oder CAB-O-SIL® oder Bentoniten auf die Oberfläche von Pulvern oder Granulaten, zur Konfektionierung absorbierender Polymerer ist ebenfalls bekannt. So lehren die EP-A-0 450 923, EP-A-0 450 922, DE-A-3 523 617, US-A-5 140 076 und US-A-4 734 478 den Zusatz von Silica bei dem Prozeß der Oberflächennachvernetzung von trockenen Pulvern absorbierender Polymere mit carboxylgruppenreaktiven Vernetzersubstanzen. Ferner beschreibt die US-4 286 082 Mischungen von Silica mit absorbierenden Polymeren für den Einsatz in Hygieneartikeln. Die JP 65 133 028A und JP 61 017 542B beschreiben Abmischungen von hydrophoben Silica Typen mit absorbierenden Polymeren. Die EP-A-0 341 951, US-A-4 990 338 und US-A-5 035 892 beschreiben den Einsatz von Silica bei der Herstellung antimikrobiell ausgerüsteter absorbierender Polymere. In der US-A-4 535 098 und EP-A-0 227 666 ist schließlich der Zusatz von kolloidalen Trägersubstanzen auf Basis Silica zur Steigerung der Gelstärke absorbierender Polymere beschrieben.

Durch diese beschriebenen "Trocken"-Abmischungen, bei denen die Zusatzstoffe lediglich auf der Oberfläche des Polymer haften, wird jedoch das Eigenschaftsprofil der absorbierenden Hydrogele verändert, z.B. werden sie hydrophiliert oder hydrophobiert, wodurch vorrangig die Flüssigkeitsaufnahmegeschwindigkeit beeinflußt wird. Zusätzlich wird dadurch zum Teil auch die Gelfestigkeit der gequollenen Partikel verstärkt, jedoch ist allen diese Polymeren gemeinsam, daß die Flüssigkeitsdurchlässigkeit (Permeabilität) durch gequollenes Gel, unabhängig von der Akquisitionszeit unbefriedigend ist.

Die Aufgabe der vorliegenden Erfindung war es daher, neue Hydrogele herzustellen, welche sich insbesondere durch eine verbesserte mechanische Stabilität, sowie eine gesteigerte Flüssigkeits-Permeabilität der gequollenen Gelteilchen auszeichnen. Diese Aufgabe sollte ohne eine der üblichen Vernetzersubstanzen erfüllt werden.

Diese Aufgabe wird überraschenderweise durch die Verwendung von Salzen der Kieselsäure gelöst, die vor, während oder nach der Polymerisationsreaktion, aber noch vor der Trocknung der Hydrogele diesen zugesetzt werden.

Gegenstand der vorliegenden Erfindung sind wäßrige Flüssigkeiten absorbierende Hydrogele, hergestellt durch Polymerisation von olefinisch ungesättigten Carbonsäuren oder deren Derivaten, dadurch gekennzeichnet, daß der Polymerisations-Reaktionsmischung vor, während oder nach der Polymerisationsreaktion und vor der Trocknung ein Alkalisalz der Kieselsäure der allgemeinen Formel I

$$M_2O \times n\ SiO_2 \qquad (I),$$

wobei M ein Alkalimetall bedeutet, und n im allgemeinen eine Zahl zwischen 0,5 bis 4 ist, zugesetzt wird, und das so erhaltene Hydrogel danach bei erhöhter Temperatur getrocknet wird.

Die Verbindungen der Formel I werden vorzugsweise in Mengen von 0,05 bis 100, besonders bevorzugt 1 bis 70, insbesondere 1 bis 40, speziell 1 bis 20 Gew.-%, berechnet auf $SiO_2$, bezogen auf das Gesamtmonomerengewicht eingesetzt, d.h. daß 100 Gew.-% Einsatz von Verbindungen der Formel I den Einsatz gleicher Gewichtsmengen Monomerer und Silikate bedeutet. M bedeutet vorzugsweise Natrium oder Kalium.

Die Herstellung dieser Alkalisilicate ist allgemein bekannt und erfolgt durch Umsetzung von wäßrigem Alkali mit $SiO_2$ oder durch Zusammenschmelzen von Quarzsand mit Alkalicarbonaten bei hohen Temperaturen im Molverhältnis 1:2 bis 4:1. Die abgekühlten glasigen Schmelzen sind in Wasser löslich und werden deshalb auch als "Wassergläser" bezeichnet.

Die im Handel erhältlichen wäßrigen Lösungen der Alkalisilicate gemäß Formel I werden durch Auflösen der festen Schmelzen in überhitztem Wasser unter Druck gewonnen.

Die wäßrigen Lösungen der Alkalisilicate (Wassergläser) reagieren infolge teilweiser Hydrolyse alkalisch. Sie enthalten neben Alkali- und Hydroxid-Ionen auch Monosilicat-Ionen, $HSiO_4^{3-}$, $H_2SiO_4^{2-}$ und $H_3SiO_4^{-}$, sowie cyclische und raumvernetzte Polysilicat-Ionen.

Beim Ansäuern wäßriger Alkalisilicatlösungen entstehen kugelförmig aufgebaute amorphe Polykieselsäuren, sogenannte Kieselsole, welche leicht zu einer gallertartigen Masse erstarren (Kiesel-Hydrogele). In ihr liegt ein durch zahlreiche wassergefüllte Poren durchsetztes Polykondensat kugelförmiger Polykieselsäuren vor. Aus dem Hydrogel erhält man durch Trocknen bei höheren Temperaturen feste Kieselgele, als besondere Form davon "Kiesel-Aerogele".

Als olefinisch ungesättigte Carbonsäuren oder deren Derivate kommen insbesondere Acrylsäure, Methacrylsäure, Crotonsäure, 2-Acrylamido-2-methylpropansulfonsäure und -phosphonsäure, Vinylphosphonsäure, Vinylphosphonsäurehalbester, deren Salze, Acrylamid, N-Vinylamide oder Gemische davon in Frage. Bevorzugt sind Acrylsäure und deren Salze.

Die Herstellung und Verwendung derartiger zur Hydrogelbildung befähigter Polymere ist in zahlreichen Patentschriften wie der EP-A-0 316 792, EP-A-0 400 283, EP-A-0 343 427, EP-A-0 205 674 und DE-A-4 418 818 beschrieben.

Die Polymerisation wird bevorzugt in homogener Phase z.B. in wäßriger Lösung als sogenannte Gelpolymerisation durchgeführt.

Die Polymerisation kann wie allgemein bekannt durch Radikalbildner wie zum Beispiel organische oder anorganische Peroxide sowie Azoverbindungen ausgelöst werden. Beispiele sind Benzoylperoxid, tert. Butylhydroperoxid, Cumolhydroperoxid, $(NH_4)_2S_2O_8$, $K_2S_2O_8$, $H_2S_2O_8$, $H_2O_2$ oder Azo-diisobutyronitril. Auch Redoxsysteme eignen sich in hervorragender Weise als Polymerisationsinitiatoren.

Die Polymerisation kann schließlich auch durch energiereiche Strahlung ausgelöst werden.

Vorzugsweise werden die sauren Polymerisate nach der Polymerisation mit Mischungen aus Alkalisilikaten und Alkalihydroxiden, bevorzugt in Form deren wäßriger Lösungen, neutralisiert. Ebenfalls bevorzugt werden die sauren Polymerisate mit Mischungen aus Alkalisilikaten und Alkalicarbonaten neutralisiert.

Vorzugsweise werden die sauren Polymerisate auf pH-Werte zwischen 3,5 und 9,0, insbesondere auf 4,0 und 6,5 neutralisiert.

Die Trocknungstemperaturen für die nachfolgende Trocknung der Hydrogele liegen vorzugsweise im Bereich zwischen 40°C und 300°C, insbesondere zwischen 120°C und 220°C.

Bei einem pH-Wert des Polymeren von 5,0 bis 9,0 liegt die Gelpermeabilität, gemessen als GLP, vorzugsweise mindestens bei

$25\times10^{-7}$ $cm^3sec/g$, besonders bevorzugt mindestens bei

$45\times10^{-7}$ $cm^3sec/g$ und insbesondere mindestens bei $60\times10^{-7}$ cm3sec/g.

Bei einem pH-Wert des Polymeren von kleiner 5,0 beträgt die Gelpermeabilität gemessen als GLP insbesondere mindestens $4\times10^{-7}$ $cm^3sec/g$, bevorzugt mindestens $10\times10^{-7}$ $cm^3sec/g$ und besonders bevorzugt mindestens $20\times10^{-7}$ $cm^3sec/g$.

Die erfindungsgemäßen Hydrogele eignen sich in hervorragender Weise als Absorbentien für wäßrige Flüssigkeiten, beispielsweise zur Absorption wäßriger Lösungen, Dispersionen und Emulsionen, insbesondere zur Absorption von Körperflüssigkeiten wie Blut und Urin, zur Herstellung von Artikeln zur Absorption wäßriger Flüssigkeiten und zur Herstellung absorbierender Hygieneartikel.

Für die Verwendung als sogenannte "Super Absorbing Polymers" (SAP) zum Einsatz von Hygieneartikeln, beispielsweise Windeln, Tampons oder Damenbinden, eignen sich insbesondere erfindungsgemäße Hydrogele auf Basis Acrylsäure, wobei diese teilweise als Alkali- oder Aminsalz vorliegen können. Die Neutralisation erfolgt erfindungsgemäß unter Zusatz von Alkali-Silikaten.

Durch Nachvernetzung, insbesondere in der Oberfläche mit Mono-, Bis- und Polyoxazolidinonen, mit dem cyclischen Ester aus Propandiol mit Kieselsäure der Formel II

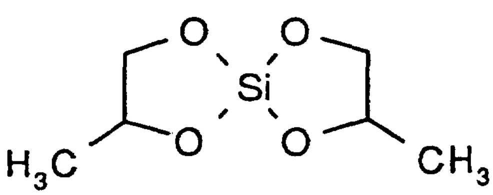

(II)

oder mit Verbindungen, welche mindestens 2 carboxylgruppenreaktive funktionelle Gruppen im Molekül enthalten, wie Di-, Tri-, oder Polyepoxide, z.B. Ethylenglykoldiglycidylether oder Haloepoxiverbindungen oder Polyaminverbindungen sowie mehrwertige Alkohole wie Ethylenglykol, Propylenglykol, Trimethylolpropan, kann die Wirkung hinsichtlich Absorption unter Druck wesentlich verbessert werden.

Das erfindungsgemäße Polymer hat im gequollenen Zustand einen härteren, stärker kristallinen Charakter, was zum einen ein Aneinanderkleben unterdrückt und woraus zum anderen eine verbesserte Flüssigkeitsweiterleitung oder Drainage, insbesondere unter Gewichtsbelastung resultiert.

In den folgenden Beispielen werden die Herstellung und Eigenschaften der erfindungsgemäßen Polymerisate erläutert und in dem Kapitel Prüfmethoden werden die Vorschriften zur Bestimmung der Eigenschaften der Hydrogele beschrieben.

Prüfmethoden

Zentrifugenretentionskapazität (CRC) :

Bei dieser Methode wird die freie Quellbarkeit des Hydrogels im Teebeutel bestimmt. Es werden 0,2000 ± 0,0050 g getrocknetes Hydrogel in einen Teebeutel eingeschweißt (Format: 60 mm x 60 mm, Dexter 1234T-Papier) und für 30 Minuten in einer 0,9 gew.-%igen Kochsalzlösung eingeweicht. Anschließend wird der Teebeutel 3 min in einer handelsüblichen Wäschezentrifuge (1400 U/min, Korbdurchmesser 230 mm) geschleudert. Die Bestimmung der aufgenommenen Flüssigkeitsmenge geschieht durch Auswägen des zentrifugierten Teebeutels.

Zur Berücksichtigung der Aufnahmekapazität des Teebeutels selbst läßt man einen Teebeutel ohne wasserabsorbierendes Hydrogel als sogenannten Blindwert mitlaufen.

$$\text{Retention CRC [g/g]} = (\text{Auswaage} - \text{Blindwert} - \text{Einwaage})/\text{Einwaage}$$

wobei

Auswaage das Naßgewicht des mit Probe gequollenen und zentrifugierten Teebeutels mit Inhalt

Einwaage das Probengewicht trocken und

Blindwert das Naßgewicht des leeren Teebeutels nach Zentrifugieren ist.

Absorption unter Druck:

Bei der Absorption unter Druck werden 0,1600 ± 0,0050 g trockenes Hydrogel gleichmäßig auf dem Siebboden einer Meßzelle verteilt. Die Meßzelle besteht aus einem Plexiglaszylinder (Höhe = 33 mm, Durchmesser = 25 mm), auf den als Boden ein Sieb aus Stahlgewebe (Maschenweite 36 micron) aufgeklebt ist.

Über das gleichmässig verteilte Hydrogel wird eine Abdeckplatte gelegt und mit einem entsprechenden Gewicht belastet. Die Zelle wird in eine Petrischale (Höhe = 10 mm, Durchmesser = 100 mm) gestellt, welche 13 ml 0,9 gew.-%ige Kochsalzlösung enthält. Man lässt das Hydrogel für 60 min die Salzlösung absorbieren. Dann nimmt man die komplette Zelle mit dem gequollenen Gel aus der Petrischale heraus und wägt die Apparatur nach Entfernen des Gewichts zurück.

Die Absorption unter Druck (AUL = Absorbency under load) wird wie folgt berechnet:

$$\text{AUL [g/g]} = (Wb - Wa) / Ws$$

wobei

Wb die Masse der Apparatur + Gel nach dem Quellen ist,

Wa die Masse der Apparatur + Einwaage vor dem Quellen ist,

Ws die Einwaage an trockenem Hydrogel ist.

Die Apparatur besteht aus Messzylinder + Abdeckplatte.

Permeabilität (GLP) :

Die Permeabilität einer gequollenen Gelschicht unter Druckbelastung von 0,3 psi wird, wie in der EP-A-0 640 330 beschrieben, als Gel-Layer-Permeabilty (GLP) einer gequollenen Gelschicht aus superabsorbierendem Polymerisat bestimmt, wobei die in zuvor genannter Patentschrift auf Seite 19 und in Figur 8 beschriebene Apparatur dahingehend modifiziert wurde, daß die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Durchbohrungen enthält. Die Vorgehensweise sowie Auswertung der Prüfmethode bleibt unverändert gegenüber der Beschreibung in der EP-A-0 640 330 und der DE-A-195 43 366. Der Durchfluß (g NaCl-Lösung/sec) wird automatisch in bestimmten Zeitintervallen erfaßt.

$$GLP = (F_g(t=0)*L_0)/(d*A*WP)(cm^3*sec/g).$$

Wobei ($F_g$(t=0) den Durchfluß der NaCl-Lösung in g/sec, der anhand einer linearen Regressionsanalyse der Daten $F_g$(t) der Durchflußbestimmungen durch Extrapolation gegen t=0 erhalten wird, $L_0$ die Dicke der Gelschicht in cm, d die Dichte der NaCl-Lösung in g/$cm^3$, A die Fläche der Gelschicht in $cm^2$ und WP den hydrostatischen Druck über der Gelschicht in dyn/$cm^2$ darstellt.

Beispiel 1

Unter adiabatischen Bedingungen werden in einem 2 l zylindrischen Weithalsreaktionskolben 1080 g auf 15°C abgekühltes vollentsalztes Wasser vorgelegt und 430 g Acrylsäure sowie 3,4 g Tetraallyloxyethan darin gelöst. Es wird Stickstoff in die Monomerlösung eingeleitet (ca. 2 l/min. für ca. 20 min.), um den Sauerstoffgehalt zu erniedrigen. Bei einem Gehalt von 1,5 ppm $O_2$ werden 7,7 g einer 10 gew.-%igen wäßrigen Lösung von 2,2'-Azobis(2-amidinopropan)-dihydrochlorid zugegeben, nach weiterem $N_2$-Einleiten und einem $O_2$-Gehalt von 1,3 ppm werden 2,6 g einer 1 gew.-%igen $H_2O_2$-Lösung zugegeben und schließlich bei einem $O_2$-Gehalt von 1,0 ppm werden 6,4 g einer 0,1 gew.-%igen Ascorbinsäurelösung zugegeben. Durch einsetzende Polymerisation, in deren Verlauf die Temperatur bis auf ca. 75°C ansteigt, entsteht ein festes Gel, das anschließend mechanisch zerkleinert wird. 1000 g des zerkleinerten Gels werden mit 10 g Natron-Wasserglas (27 gew.-%ig bez $SiO_2$ und 14 gew.-%ig bez. NaOH), gelöst in 228,2 g Natronlauge 50 gew.-%ig versetzt (Neutralisationsgrad der Acrylsäure 74 Mol-%), zweimal durch einen Mischextruder gefahren und die entstandenen Gelpartikel bei Temperaturen über 150°C getrocknet, gemahlen und gesiebt.

Man erhält ein Produkt, im wesentlichen u.a. gekennzeichnet durch folgende physikalische Daten, alle gemessen in NaCl 0,9 Gew.-%: Extrahierbare Anteile (1 h-Wert) 2,1 %, Absorption unter Druck AUL (20 g/$cm^2$) = 20,9 g/g, Permeabilität (GLP) = 3 ($\times 10^{-7}$ $cm^3$sec/g). 100 g des so erhaltenen Produktes wurden in einem Pulvermischaggregat mit 10g einer homogenen Lösung, bestehend aus 3,0 g Methanol, 7,0 g Wasser und 0,1g Ethylenglykoldiglycidylether besprüht und während eines Zeitraumes von 40 Min. bei einer Temperatur von 140°C getempert.

Es wurde ein Produkt mit folgenden Kenndaten (gemessen in 0,9 %iger wäßriger NaCl-Lösung) erhalten:

Zentrifugenretention: 33,1 g/g
AUL (60g/cm2) : 24,7 g/g
GLP : 60 ($\times 10^{-7}$ $cm^3$sec/g).

Vergleichsbeispiel 1

Die Polymerisation verläuft vollkommen analog zu Beispiel 1, jedoch wird bei der Aufarbeitung zu 1000 g des zerkleinerten Gels keine Wasserglaslösung eingesetzt, stattdessen erfolgt die Neutralisation ausschließlich mit 168 g 50 gew.-%iger Natronlauge. Man erhält ein Produkt, das sich dadurch von dem Produkt aus Beispiel 1 unterscheidet, daß es keinerlei Permeabilität aufweist, d.h. keine Flüssigkeit durch die gequollene Gelschicht durchläßt und die GLP somit einen Wert von 0 ($10^{-7}cm^3$sec/g) aufweist. Wird dieses Produkt analog Beispiel 1 oberflächennachvernetzt, weist das nachvernetzte Produkt lediglich eine Permeabilität (GLP) von 20 ($10^{-7}cm^3$sec/g) auf.

Beispiel 2

In einem durch geschäumtes Kunststoffmaterial gut isolierten Polyethylengefäß mit einem Fassungsvermögen von 10 l werden 3500 g vollentsalztem Wasser mit einer Temperatur von 4°C vorgelegt und 1800 g Acrylsäure unter Rühren zufliessen gelassen. Es werden nun 10,8 g Pentaerythritholtriallylether zugesetzt und die Lösung durch Einleiten von Stickstoff inertisiert. Dann erfolgt die Zugabe des Initiatorsystems, bestehend aus 2,5 g 2,2'-Azobisamidinopropan-dihydrochlorid, gelöst in 20 g vollentsalztem Wasser, 4 g Kaliumperoxodisulfat, gelöst in 50 g vollentsalztem Wasser sowie 0,4 g Ascorbinsäure, gelöst in 20 g vollentsalztem Wasser nacheinander unter Rühren. Die Reaktionslösung wird ohne Rühren stehen gelassen, wobei durch die einsetzende Polymerisation, in deren Verlauf die Temperatur auf ca. 90°C ansteigt, ein festes Gel entsteht.

1000 g des so hergestellten Gels werden unter Zugabe einer Lösung von 0,96 g Natrium-Wasserglas 27 %ig (der Firma MERCK) in 216,6 g NaOH 50 %ig mechanisch zerkleinert und anschliessend nochmals in einem Mischextruder behandelt. Die entstandenen Gelpartikel werden bei Temperaturen über 150°C getrocknet und gemahlen.

100 g des so hergestellten Polymerpulvers wurden in einem Labor-Mischaggregat mit einer Lösung aus 7 ml Wasser, 3 g Methanol und 0,20 g 2-Oxazolidinon besprüht und während eines Zeitraumes von 60 Min. bei 175°C getempert. Das erhaltene Material ist gekennzeichnet durch folgende Produktdaten:

Zentrifugenretention: 24,6 g/g
AUL (60g/$cm^2$) : 23,9 g/g
GLP : 8 ($\times 10^{-7}$ $cm^3$sec/g).

Vergleichsbeispiel 2

Die Polymerisation verläuft vollkommen analog zu Beispiel 2, jedoch wird bei der Aufarbeitung zu 1000 g des zerkleinerten Gels keine Wasserglaslösung eingesetzt, stattdessen erfolgt die Neutralisation ausschließlich mit 218,2 g 50 gew.-%iger Natronlauge. Man erhält ein Produkt, das nach Oberflächennachvernetzung analog Beispiel 2 eine Permeabilität (GLP) von lediglich 4 ($\times 10^{-7}$$cm^3$sec/g) aufweist.

Beispiel 3

Es wird vollkommen analog zu Beispiel 2 polymerisiert, jedoch werden bei der Aufarbeitung 1000 g des zerkleinerten Gels mit einer Lösung aus 4,9 g Na-Wasserglas 35 %ig (27 % $SiO_2$ + 8 % $Na_2O$) in 215,2 g NaOH 50 gew.-%ig neutralisiert und getrocknet. 100 g des Polymerpulvers werden in einem Labormischaggregat mit einer Lösung von 0,01 g Sorbitanmonococoat und 0,25 g N-Methyloxazolidinon in 10 ml Wasser besprüht und während eines Zeitraumes von 45 Minuten bei 180°C getempert. Das erhaltene Produkt ist gekennzeichnet durch folgende Daten:

Zentrifugenretention CRC : 31,3 g/g
Absorption unter Druck, AUL 60 g/$cm_2$ : 21,4 g/g
Permeabilität (GLP): 4 ($\times 10^{-7}$$cm^3$sec/g)

Vergleichsbeispiel 3

Die Polymerisation verläuft vollkommen analog zu Beispiel 3, jedoch wird bei der Aufarbeitung zu 1000 g des zerkleinerten Gels keine Wasserglaslösung eingesetzt, stattdessen erfolgt die Neutralisation ausschließlich mit 221,5 g 50 gew.-%iger Natronlauge. Man erhält ein Produkt, das nach Oberflächennachvernetzung analog Beispiel 3 folgende Daten aufweist:

Zentrifugenretention CRC : 31,8 g/g
Absorption unter Druck, AUL 60 g/$cm_2$ : 20,9 g/g
Permeabilität (GLP): 1 ($\times 10^{-7}$$cm^3$sec/g)

Vergleichsbeispiel 4

Unter adiabatischen Bedingungen werden in einem 5 1 zylindrischen Weithalsreaktionskolben 2837 g auf 10°C abgekühltes vollentsalztes Wasser vorgelegt und 1040 g Acrylsäure sowie 8,3 g Pentaerythritoltriallylether darin gelöst. Es wird Stickstoff in die Monomerlösung eingeleitet (ca. 2 l/Min. für ca. 20 Min.), um den Sauerstoffgehalt zu erniedrigen. Bei einem Gehalt von 1,5 ppm $O_2$ wird eine Lösung von 0,52 g 2,2'-Azobis(2-amidiaopropan)-dihydrochlorid in 25 g vollentsalztem Wasser zugegeben, nach weiterem $N_2$-Einleiten und einem $O_2$-Gehalt von 1,3 ppm werden 12,165 g einer 0,47 %igen $H_2O_2$-Lösung zugegeben und schließlich bei einem $O_2$-Gehalt von 1,0 ppm werden 16,5 g einer 0,1 gew.-%igen Ascorbinsäurelösung zugegeben. Durch einsetzende Polymerisation, in deren Verlauf die Temperatur bis auf ca. 75°C ansteigt, entsteht ein festes Gel, das anschließend mechanisch zerkleinert wird. 1000 g des zerkleinerten Gels werden mit 216,7 g Natronlauge 50 gew.-%ig versetzt (Neutralisationsgrad der Acrylsäure 74 Mol-%), zweimal durch einen Mischextruder gefahren und die entstandenen Gelpartikel bei Temperaturen über 150°C getrocknet, gemahlen und gesiebt.

Man erhält ein Produkt, im wesentlichen u.a. gekennzeichnet durch folgende physikalische Daten, alle gemessen in NaCl 0,9 Gew.-%:

Extrahierbare Anteile (1 h-Wert) : 3,7 %,
Absorption unter Druck (AUL, 21 g/cm$^2$) : 11,1 g/g,
Zentrifugenretention (CRC) : 33,8 g/g,
Permeabilität (GLP) : 0,1 ($10^{-7}$cm$^3$sec/g).

In analoger Weise wurden jeweils 1000 g gemäß Vergleichsbeispiel 4 hergestellten Polyacrylatgels jetzt mit unterschiedlichen Mischungs-Mengen Na-Wasserglas/NaOH 50 %ig bzw. Na-Wasserglas/Alkalicarbonat neutralisiert. Verwendet wurde jeweils Natronwasserglas der Firma MERCK mit einem Gehalt von 27 Gew.-% $SiO_2$ und 8 Gew.-% $Na_2O$.

Einsatzmengen und Produktdaten der Herstellungsbeispiele 4 bis 9 sind folgender Tabelle 1 zu entnehmen:

Tabelle 1

| Beispiel | Na-Wasserglas (für 1000 g Gel) g | Alkali (für 1000 g Gel) | pH | CRC g/g | AUL (21 g/cm$^2$) g/g | GLP $10^{-7}$cm$^3$sec/g |
|---|---|---|---|---|---|---|
| Vergl. 4 | ---- | 216,7 g NaOH 50% | 5,83 | 33,8 | 11,1 | 0,1 |
| 4 | 288,9 | 124,2 g NaOH 50% | 5,65 | 17,9 | 20,5 | 10 |
| 5 | 288,9 | 110,0 g $K_2CO_3$ | 5,66 | 17,1 | 19,9 | 8 |
| 6 | 288,9 | 85,0 g $Na_2CO_3$ | 5,63 | 18,0 | 20,1 | 11 |
| 7 | 481,5 | 62,6 g NaOH 50% | 5,49 | 15,0 | 17,3 | 32 |
| 8 | 481,5 | 124,2 g NaOH 50% | 7,48 | 15,4 | 16,9 | 28 |
| 9 | 674,0 | 1,0 g NaOH 50% | 5,20 | 11,9 | 14,8 | 45 |

Vergleichsbeispiel 4a:

100 g des gemäß Vergleichsbeispiel 4 erhaltenen Produktes wurden in einem Pulvermischaggregat mit 10,42 g einer homogenen Lösung, bestehend aus 6,0 g 1,2-Propandiol, 2,3g Wasser, 2,0 g eines Polyamidoaminharzes in 15 %iger wäßriger Lösung (RESAMIN® VHW 3608 der Clariant GmbH) und 0,12 g $Al_2(SO_4)_3 \cdot 18\ H_2O$ besprüht und während eines Zeitraumes von 120 Min. bei einer Temperatur von 140°C getempert.

Es wurde ein Produkt mit folgenden Kenndaten (gemessen in 0,9 gew.-%iger wäßriger NaCl-Lösung) erhalten:

Zentrifugenretention: 28,2g/g
AUL (60g/cm$^2$) : 24,6 g/g
GLP : 20 ($10^{-7}$ cm$^3$sec/g).

In analoger Weise zu Vergleichsbeispiel 4a wurden weitere gemäß vergleichsbeispiel 4 hergestellte und mit unterschiedlichen Mischungsmengen Na-Wasserglas/NaOH 50 %ig neutralisierte Polyacrylatgele oberflächennachvernetzt.

Einsatzmengen und Produktdaten der Herstellungsbeispiele 10 bis 15 sind folgender Tabelle 2 zu entnehmen:

Tabelle 2

| Beispiel | Na-Wasserglas (für 1000 g Gel) g | NaOH 50 %ig (für 1000 g Gel) g | CRC g/g | AUL (60 g/cm$^2$) g/g | GLP $10^{-7}$cm$^3$sec/g |
|---|---|---|---|---|---|
| Vergl.4a | ---- | 216,7 | 28,2 | 24,6 | 20 |
| 10 | 9,6 | 213,6 | 27,2 | 24,0 | 35 |
| 11 | 19,3 | 210,5 | 26,7 | 23,3 | 41 |
| 12 | 28,9 | 207,4 | 24,7 | 23,2 | 52 |

Tabelle 2 (fortgesetzt)

| Beispiel | Na-Wasserglas (für 1000 g Gel) g | NaOH 50 %ig (für 1000 g Gel) g | CRC g/g | AUL (60 g/cm2) g/g | GLP $10^{-7}cm^3sec/g$ |
|---|---|---|---|---|---|
| 13 | 38,5 | 204,3 | 24,3 | 22,9 | 65 |
| 14 | 48,1 | 201,3 | 24,2 | 22,7 | 75 |
| 15 | 96,3 | 155,1 | 22,7 | 21,4 | 87 |

Beispiel 16

In einem durch geschäumtes Kunststoffmaterial gut isolierten Polyethylengefäß mit einem Fassungsvermögen von 10 l werden 3650 g vollentsalztem Wasser mit einer Temperatur von 20°C vorgelegt und 500 g Natriumhydrogencarbonat darin suspendiert. 2000 g Acrylsäure werden unter Rühren zufließen gelassen, wobei sich die Monomerlösung bis auf ca. 13°C abkühlt. Geschwindigkeitsbestimmend für die Acrylsäure-Zugabe ist die Schaumentwicklung als Folge der Freisetzung von $CO_2$. Es werden nun 3 g Sorbitanmonococoat, dispergiert in 100 g vollentsalztem Wasser sowie 8,1 g Allylmethacrylat zugesetzt und die Lösung durch Einleiten von Stickstoff inertisiert. Dann erfolgt nacheinander unter Rühren die Zugabe des Initiatorsystems, bestehend aus 1,66 g 2,2'-Azobis-amidinopropandihydrochlorid, gelöst in 20 g vollentsalztem Wasser, 3,3 g Kaliumperoxodisulfat, gelöst in 150 g vollentsalztem Wasser sowie 0,3 g Ascorbinsäure, gelöst in 25g vollentsalztem Wasser. Die Reaktionslösung wird ohne Rühren stehen gelassen, wobei durch die einsetzende Polymerisation, in deren Verlauf die Temperatur bis auf ca. 110°C ansteigt, ein festes Gel entsteht.

Je 1000 g des so hergestellten Gels werden unter Zugabe je einer Lösung von unterschiedlichen Mengen Na-Wasserglas 27 %ig (der Firma MERCK) in NaOH 50 %ig mechanisch zerkleinert und anschließend nochmals in einem Mischextruder behandelt. Die entstandenen Gelpartikel werden im Heißluftstrom einer Temperatur von 170°C getrocknet, anschließend gemahlen und gesiebt.

Einsatzmengen und Produktdaten der Herstellungsbeispiele 16 bis 19 sind folgender Tabelle 3 zu entnehmen:

Tabelle 3

| Beispiel | Na-Wasserglas (für 1000 g Gel) g | NaOH 50 %ig (für 1000 g Gel) g | pH | CRC g/g | AUL (35 g/cm2) g/g | GLP $10^{-7}cm^3sec/g$ |
|---|---|---|---|---|---|---|
| 16 (Vergl.) | ---- | 18,35 | 4,4 | 20,7 | 10,8 | 2,5 |
| 17 | 30,8 | --- | 4,3 | 17,2 | 15,4 | 14 |
| 18 | 61,7 | --- | 4,3 | 17,7 | 15,3 | 23 |
| 19 | 123,3 | --- | 4,5 | 18,7 | 15,6 | 27 |

Beispiel 20

Unter adiabatischen Bedingungen werden in einem 5 1 zylindrischen Weithalsreaktionskolben 2942 g auf 10°C abgekühltes vollentsalztes Wasser vorgelegt und 1000 g Acrylsäure sowie 4,5 g Pentaerythritoltriallylether darin gelöst. Es wird Stickstoff in die Monomerlösung eingeleitet (ca. 2 l/Min. für ca. 20 Min.), um den Sauerstoffgehalt zu erniedrigen. Bei einem Gehalt von 1,5 ppm $O_2$ wird eine Lösung von 0,52 g 2,2'-Azobis(2-amidinopropan)-dihydrochlorid in 25 g vollentsalztem Wasser zugegeben, nach weiterem $N_2$-Einleiten und einem $O_2$-Gehalt von 1,3 ppm werden 12 g einer 0,47 %igen $H_2O_2$-Lösung zugegeben und schließlich bei einem $O_2$-Gehalt von 1,0 ppm werden 16,5 g einer 0,1 gew.-%igen Ascorbinsäurelösung zugegeben. Durch einsetzende Polymerisation, in deren Verlauf die Temperatur bis auf ca. 70°C ansteigt, entsteht ein festes Gel, das anschließend mechanisch zerkleinert wird.

Je 1000 g des so hergestellten Gels werden mechanisch zerkleinert und mit NaOH (Beispiel 20) oder mit Na-Wasserglas 27 %ig (der Firma MERCK) (Beispiele 21-24) neutralisiert und anschliessend nochmals in einem Mischextruder behandelt. Die entstandenen Gelpartikel werden im Heißluftstrom einer Temperatur von 100°C getrocknet, anschließend gemahlen und gesiebt.

Einsatzmengen und Produktdaten der Herstellungsbeispiele 20 bis 24 sind folgender Tabelle 4 zu entnehmen:

Tabelle 4

| Beispiel | Na-Wasserglas (für 1000 g Gel) g | NaOH 50 %ig (für 1000 g Gel) g | pH | CRC g/g | GLP $10^{-7}cm^3sec/g$ |
|---|---|---|---|---|---|
| 20 (Vergl.) | ---- | 75 | 4,36 | 29,5 | 1 |
| 21 | 244 | --- | 4,03 | 15,3 | 12 |
| 22 | 305 | --- | 4,25 | 18,9 | 17 |
| 23 | 350 | --- | 4,36 | 19,7 | 21 |
| 24 | 400 | --- | 4,50 | 20,6 | 25 |

Beispiel 25

Unter adiabatischen Bedingungen werden in einem 5 l zylindrischen Weithalsreaktionskolben 2840 g auf 10°C abgekühltes vollentsalztes Wasser vorgelegt, 77,0 g Natronwasserglas 35 %ig der Fa. MERCK (27 Gew.-% $SiO_2$ + 8 Gew.-% $Na_2O$) und 1040 g Acrylsäure sowie 10,4 g Pentaerythritoltriallylether darin gelöst. Wasserglas und Acrylsäure müssen langsam und in der richtigen Reihenfolge zugegeben werden, um ein Ausfällen des Na-Silikats zu vermeiden. Es wird Stickstoff in die Monomerlösung eingeleitet (ca. 2 l/Min. für ca. 20 Min.), um den Sauerstoffgehalt zu erniedrigen. Bei einem Gehalt von 1,5 ppm $O_2$ wird eine Lösung von 0,52 g 2,2'-Azobis(2-amidinopropan)-dihydrochlorid in 25 g vollentsalztem Wasser zugegeben, nach weiterem $N_2$-Einleiten und einem $O_2$-Gehalt von 1,3 ppm werden 12,165 g einer 0,47 %igen $H_2O_2$-Lösung zugegeben und schließlich bei einem $O_2$-Gehalt von 1,0 ppm werden 16,0 g einer 0,1 %igen Ascorbinsäurelösung zugegeben. Durch einsetzende Polymerisation, in deren Verlauf die Temperatur bis auf ca. 75°C ansteigt, entsteht ein festes Gel, das anschließend mechanisch zerkleinert wird. 1000 g des zerkleinerten Gels werden mit 198 g Natronlauge 50 gew.-%ig versetzt, zweimal durch einen Mischextruder gefahren und die entstandenen Gelpartikel auf einem Walzentrockner bei Temperaturen von ca. 180°C Trockneroberfläche getrocknet, gemahlen und gesiebt.

Man erhält ein Produkt, im wesentlichen u.a. gekennzeichnet durch folgende physikalische Daten, alle gemessen in NaCl 0,9 %:

Absorption unter Druck : (AUL, 21 $g/cm^2$) : 21,6 g/g,
Zentrifugenretention (CRC) : 30,0 g/g.

100 g des erhaltenen Produktes wurden in einem Pulvermischaggregat mit 10,00 g einer homogenen Loesung, bestehend aus 3,83 g 1,2-Propandiol, 4,05 g Wasser, 2,0 g eines Polyamidoaminharzes in 15 %iger waessriger Lösung (RESAMIN VHW 3608® der CLARIANT GmbH) und 0,12 g $Al_2(SO_4)_3 \cdot 18\ H_2O$ besprüht und während eines Zeitraumes von 120 Min. bei einer Temperatur von 140°C getempert.

Es wurde ein Produkt erhalten, gekennzeichnet durch folgende physikalische Daten, alle gemessen in NaCl 0,9 %.

Zentrifugenretention: 26 g/g
AUL (60$g/cm^2$) : 24 g/g
GLP : 62 ($10^{-7}$ $cm^3sec/g$).

Vergleichsbeispiel 25

Vollkommen analog zu Beispiel 25 wird polymerisiert, mit dem Unterschied, daß kein Natronwasserglas verwendet wird und stattdessen bei der anschließenden Neutralisation für 1000 g Polymergel jetzt 214 g NaOH 50 gew.-%ig verwendet werden. Auch Trocknung und Mahlung sind identisch zu Beispiel 25.

Man erhält ein Produkt, im wesentlichen u.a. gekennzeichnet durch folgende physikalische Daten, alle gemessen in NaCl 0,9 %:

Absorption unter Druck (AUL, 21 $g/cm^2$) : 12,0 g/g
Zentrifugenretention (CRC) : 32,8 g/g.

100 g des erhaltenen Produktes wurden ebenfalls vollkommen analog zu Beispiel 25 oberflächennachvernetzt, wobei ein Produkt erhalten wurde, gekennzeichnet durch folgende physikalische Daten, alle gemessen in NaCl

0,9 %.

Zentrifugenretention: 28 g/g
AUL (60g/$cm^2$) : 24 g/g
GLP : 33 ($10^{-7}$ $cm^3$sec/g).

## Patentansprüche

1. Wäßrige Flüssigkeiten absorbierende Hydrogele, hergestellt durch Polymerisation von olefinisch ungesättigten Carbonsäuren oder deren Derivaten, **dadurch gekennzeichnet, daß** der Polymerisations-Reaktionsmischung vor, während oder nach der Polymerisationsreaktion und vor der Trocknung ein Alkalisalz der Kieselsäure der allgemeinen Formel I

$$M_2O \times n\ SiO_2 \quad (I),$$

wobei M ein Alkalimetall bedeutet, und n eine Zahl zwischen 0,5 bis 4 ist, zugesetzt wird, und das so erhaltene Hydrogel danach bei erhöhter Temperatur getrocknet wird.

2. Polymere nach Anspruch 1, **dadurch gekennzeichnet, daß** sie mit Alkalisilicaten in Mengen von 0,05 Gew.-% bis 100 Gew.-%, berechnet auf $SiO_2$, bezogen auf das Gesamtmonomergewicht, versetzt werden.

3. Polymere nach Anspruch 1, **dadurch gekennzeichnet, daß** sie mit Alkalisilicaten in Mengen von 1 Gew.-% bis 70 Gew.-%, berechnet auf $SiO_2$, bezogen auf das Gesamtmonomergewicht, versetzt werden.

4. Polymere nach den Ansprüchen 1 bis 3, **dadurch gekennzeichnet, daß** die sauren Polymerisate mit Mischungen aus Alkalisilikaten und Alkalihydroxiden neutralisiert werden.

5. Polymere nach den Ansprüchen 1 bis 3, **dadurch gekennzeichnet, daß** die sauren Polymerisate mit Mischungen aus Alkalisilikaten und Alkalicarbonaten neutralisiert werden.

6. Polymere nach den Ansprüchen 1 bis 5, **dadurch gekennzeichnet, daß** die sauren Polymerisate auf pH-Werte von 3,5 bis 9,0 neutralisiert werden.

7. Polymere nach den Ansprüchen 1 bis 6, **dadurch gekennzeichnet, daß** die Trocknungstemperaturen im Bereich von 40°C bis 300°C liegen.

8. Polymere nach den Ansprüchen 1 bis 7, **dadurch gekennzeichnet, daß** bei einem pH-Wert des Polymeren von 5,0 bis 9,0 die Gelpermeabilität gemessen als GLP mindestens $25 \times 10^{-7}$ $cm^3$sec/g beträgt.

9. Polymere nach den Ansprüchen 1 bis 7, **dadurch gekennzeichnet, daß** bei einem pH-Wert des Polymeren von kleiner 5,0 die Gelpermeabilität gemessen als GLP mindestens $4 \times 10^{-7}$ $cm^3$sec/g beträgt.

10. Verfahren zur Herstellung der Polymere gemäß Anspruch 1, indem man der Polymerisationsmischung der Polymerisation von olefinisch ungesättigten Carbonsäuren oder deren Derivaten vor, während oder nach der Polymerisationsreaktion und vor der Trocknung ein Alkalisalz der Kieselsäure der Formel I zusetzt und das so erhaltene Hydrogel danach bei erhöhter Temperatur trocknet.

11. Verwendung der Polymere gemäß Anspruch 1 zur Absorption wäßriger Lösungen, Dispersionen und Emulsionen.

12. Verwendung der Polymere gemäß Anspruch 1 zur Herstellung von Artikeln zur Absorption wäßriger Flüssigkeiten.

## Claims

1. Hydrogels capable of absorbing aqueous fluids, prepared by polymerization of olefinically unsaturated carboxylic

acids or derivatives thereof, wherefor the polymerization reaction mixture is admixed with an alkali metal silicate of the general formula I

$$M_2O \times n\ SiO_2 \quad (I),$$

where M is an alkali metal and n is from 0.5 to 4, before, during or after the polymerization reaction and before drying and the hydrogel thus obtained is then dried at elevated temperature.

**2.** Polymers as claimed in claim 1, admixed with alkali metal silicates in amounts of from 0.05% by weight to 100% by weight, reckoned on $SiO_2$ and based on the total monomer weight.

**3.** Polymers as claimed in claim 1, admixed with alkali metal silicates in amounts of from 1% by weight to 70% by weight, reckoned on $SiO_2$ and based on the total monomer weight.

**4.** Polymers as claimed in any of claims 1 to 3, wherefor the acidic addition polymers are neutralized with mixtures of alkali metal silicates and alkali metal hydroxides.

**5.** Polymers as claimed in any of claims 1 to 3, wherefor the acidic addition polymers are neutralized with mixtures of alkali metal silicates and alkali metal carbonates.

**6.** Polymers as claimed in any of claims 1 to 5, wherefor the acidic addition polymers are neutralized to a pH of from 3.5 to 9.0.

**7.** Polymers as claimed in any of claims 1 to 6, wherefor the drying temperatures are in the range from 40°C to 300°C.

**8.** Polymers as claimed in any of claims 1 to 7, **characterized by** a GLP gel permeability of not less than $25 \times 10^{-7}$ $cm^3$sec/g at a polymer pH of from 5.0 to 9.0.

**9.** Polymers as claimed in any of claims 1 to 7, **characterized by** a GLP gel permeability of not less than $4 \times 10^{-7}$ $cm^3$sec/g at a polymer pH of less than 5.0.

**10.** The process for preparing the polymers of claim 1 by admixing the polymerization mixture of the polymerization of olefinically unsaturated carboxylic acids or derivatives thereof with an alkali metal silicate of the formula I before, during or after the polymerization reaction and before drying and then drying the thus obtained hydrogel at elevated temperature.

**11.** Use of the polymers of claim 1 for absorbing aqueous solutions, dispersions and emulsions.

**12.** Use of the polymers of claim 1 for producing articles for absorbing aqueous fluids.

## Revendications

**1.** Hydrogels absorbant des liquides aqueux, préparés par polymérisation d'acides carboxyliques oléfiniquement insaturés ou de leurs dérivés, **caractérisés en ce que**, au mélange réactionnel de la polymérisation, on ajoute, avant, pendant ou après la réaction de polymérisation et avant le séchage, un sel de métal alcalin de l'acide silicique répondant à la formule générale I :

$$M_2O \times n\ SiO_2 \quad (I)$$

dans laquelle M représente un métal alcalin et n est un nombre entre 0,5 et 4, et **en ce qu'**on sèche ensuite à la température ambiante l'hydrogel ainsi obtenu.

**2.** Polymères suivant la revendication 1, **caractérisés en ce qu'**ils sont additionnés de silicates de métal alcalin en des quantités de 0,05% en poids à 100% en poids, exprimés en $SiO_2$, par rapport au poids total de monomère.

**3.** Polymères suivant la revendication 1, **caractérisés en ce qu'**ils sont additionnés de silicate de métal alcalin en des quantités de 1% en poids à 70% en poids, exprimés en $SiO_2$, par rapport au poids total de monomère.

**4.** Polymères suivant l'une des revendications 1 à 3, **caractérisés en ce que** les polymères acides sont neutralisés par des mélanges de silicates de métal alcalin et d'hydroxydes de métal alcalin.

**5.** Polymères suivant l'une des revendications 1 à 3, **caractérisés en ce que** les polymères acides sont neutralisés par des mélanges de silicates de métal alcalin et de carbonates de métal alcalin.

**6.** Polymères suivant l'une des revendications 1 à 5, **caractérisés en ce que** les polymères acides sont neutralisés à des valeurs de pH de 3,5 à 9,0.

**7.** Polymères suivant l'une des revendications 1 à 6, **caractérisés en ce que** les températures de séchage se situent dans la gamme de 40°C à 300°C.

**8.** Polymères suivant l'une des revendications 1 à 7, **caractérisés en ce que**, à une valeur de pH du polymère de 5,0 à 9,0, la perméabilité du gel, mesurée comme GLP, est d'au moins $25x10^{-7}$ $cm^3s/g$.

**9.** Polymères suivant l'une des revendications 1 à 7, **caractérisés en ce que**, à une valeur de pH du polymère inférieure à 5,0, la perméabilité du gel, mesurée comme GLP, est d'au moins $4x10^{-7}$ $cm^3s/g$.

**10.** Procédé de préparation des polymères suivant la revendication 1, dans lequel on ajoute au mélange de la polymérisation d'acides carboxyliques oléfiniquement insaturés ou de leurs dérivés, avant, pendant ou après la réaction de polymérisation et avant le séchage, un sel de métal alcalin de l'acide silicique de la formule I et on sèche ensuite à température élevée l'hydrogel ainsi obtenu.

**11.** Utilisation des polymères suivant la revendication 1, pour l'absorption de solutions, dispersions et émulsions aqueuses.

**12.** Utilisation des polymères suivant la revendication 1, pour la préparation d'articles destinés à l'absorption de liquides aqueux.